(19) Europäisches Patentamt
European Patent Office
Office européen des brevets

(11) Publication number: **0 056 172**
**B1**

# (12) EUROPEAN PATENT SPECIFICATION

(45) Date of publication of patent specification: **03.04.85**

(21) Application number: **81201386.0**

(22) Date of filing: **18.12.81**

(51) Int. Cl.4: **C 07 C 59/90,** C 07 C 59/68, C 07 C 51/09, C 07 C 51/41, C 07 C 149/40, C 07 C 148/00, A 61 K 31/19 // C07C69/736, C07C69/738, C07C69/732, C07C67/343, C07C67/303, C07C67/333, C07C149/36, C07C49/84, C07C49/825, C07C45/63, C07C45/67, C07C45/68

## (54) Phenoxy- and thiophenoxy compounds, methods for their preparation and pharmaceutical formulations containing them.

(30) Priority: **09.01.81 GB 8100568**
**09.01.81 GB 8100569**

(43) Date of publication of application:
**21.07.82 Bulletin 82/29**

(45) Publication of the grant of the patent:
**03.04.85 Bulletin 85/14**

(84) Designated Contracting States:
**AT BE CH DE FR GB IT LI LU NL SE**

(56) References cited:
**EP-A-0 028 063**
**DE-A-1 911 539**
**FR-A-2 147 287**
**FR-A-2 241 296**
**FR-A-2 294 691**
**FR-A-2 354 311**
**GB-A-1 007 820**
**GB-A-1 405 254**

(73) Proprietor: **FISONS plc**
**Fison House Princes Street**
**Ipswich Suffolk IP1 1QH (GB)**

(72) Inventor: **Hardern, David Norman**
**6 Charnwood Fields Sutton Bonington**
**Loughborough Leicestershire (GB)**
Inventor: **Lee, Thomas Brian**
**51 Outwoods Road**
**Loughborough Leicestershire (GB)**
Inventor: **Bantick, John Raymond**
**6 Huntingdon Close Burton on the Wolds**
**Loughborough Leicestershire (GB)**

(74) Representative: **Craig, Christopher Bradberry et al**
**Fisons plc 12 Derby Road**
**Loughborough Leicestershire LE11 0BB (GB)**

**EP 0 056 172 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention concerns new compounds, processes for their preparation and compositions containing them.

According to the invention we provide compounds of formula I,

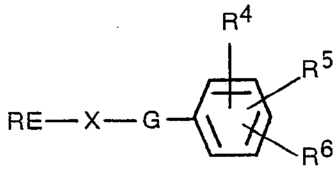

I

in which R is a group of formula II,

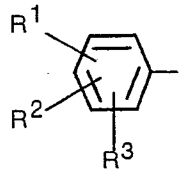

II

$R^1$, $R^2$ and $R^3$, which may be the same or different each represent hydrogen, alkyl, alkenyl, alkenyloxy, halogen, alkoxy, alkanoyl or hydroxy,

one or more of $R^4$, $R^5$ and $R^6$ represents alkyl, alkanoyl, alkenyl, —COOH or —ACOOH where A represents Y, OY or SY and Y represents a —CH=CH—, methylene, ethylene or 1,3-propylene chain, and the remainder of $R^4$, $R^5$ and $R^6$ represent hydrogen,

X represents a hydrocarbon chain having from 2 to 7 carbon atoms optionally substituted by hydroxy,

E represents —S—, —O—, or —$CH_2$—, and

G represents —S— or —O—,

provided that

a) when $R^1$ and $R^2$ are hydrogen $R^3$ does not represent chlorine, isopropyl or t-butyl and is not para to E, or

b) when E and G are both —O—, either

i) the R group is not of formula III,

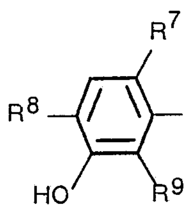

III

in which

$R^7$ is hydrogen or alkyl,

$R^8$ is alkanoyl, and

$R^9$ is alkyl or alkenyl,

when only one of $R^4$, $R^5$ and $R^6$ represent a group —COOH, —Y—COOH or —OYCOOH, or

when one of $R^4$, $R^5$ and $R^6$ represent a group —COOH, —Y—COOH or —OYCOOH and another of $R^4$, $R^5$ and $R^6$ represents —COOH, or

ii) two or more of $R^4$, $R^5$ and $R^6$ represent —ACOOH, or

iii) at least one or $R^4$, $R^5$ and $R^6$ represents a group —SYCOOH, or

iv) none of $R^4$, $R^5$ and $R^6$ represents a group selected from —COOH and —ACOOH,

c) when one of $R^4$, $R^5$ and $R^6$ represents —COOH, —$CH_2COOH$, —$(CH_2)_2$ COOH or —$(CH_2)_3$ COOH para to G, another of $R^4$, $R^5$ and $R^6$ is hydrogen or alkyl, the third of $R^4$, $R^5$ and $R^6$ is hydrogen, $R^1$ and $R^2$ are selected from hydrogen, alkyl, alkoxy and halogen, and $R^3$ is other than hydrogen, then X carries a hydroxy substituent, or

d) when $R^4$ represents $—OCH_2COOH$, $R^5$ and $R^6$ are both hydrogen, $R^1$ is hydrogen and $R^2$ and $R^3$ are selected from alkyl, alkoxy and halogen, then X carries a hydroxy substituent,

and pharmaceutically acceptable derivatives of those compounds containing an acidic function.

The invention also provides the compounds of formula I and pharmaceutically acceptable derivatives thereof, as pharmaceuticals.

According to the invention we also provide a process for the preparation of a compound of formula I or a pharmaceutically acceptable derivative thereof, which comprises,

(a) producing a compound of formula I in which one or more of $R^4$, $R^5$ and $R^6$ represents —COOH or —ACOOH, by selective hydrolysis of a compound of formula IV,

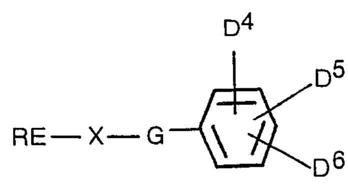

IV

in which R, E, X and G are as defined above,

one or more of $D^4$, $D^5$ and $D^6$ represent a group hydrolysable to —COOH, or to —ACOOH, and the remainder of $D^4$, $D^5$ and $D^6$ represent groups $R^4$, $R^5$ and $R^6$ as defined above,

(b) reacting a compound of formula REZ with a compound of formula V or an ester thereof,

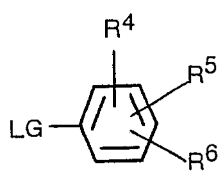

V

in which

R, E, G, $R^4$, $R^5$ and $R^6$ are as defined above, and

L and Z represent the pair of groups (i) hydrogen or a reactive metal, and (ii) a hydrocarbon chain having from 2 to 7 carbon atoms and carrying an anion forming group or an epoxide group,

(c) producing a compound of formula I in which one or more of $R^1$ to $R^6$ represent an alkyl group containing at least 2 carbon atoms and/or A represents an ethylene chain, by hydrogenation of a compound of formula I wherein one or more of $R^1$ to $R^6$ represents an alkenyl or alkanoyl group, and/or A represents —CH=CH—,

(d) producing a compound of formula I in which one or more of $R^4$, $R^5$ and $R^6$ represent —ACOOH where A represents a group OY or SY, by reacting a corresponding compound of formula I in which either an adjacent pair of $R^1$, $R^2$ and $R^3$ represent —OH and an alkanoyl group, or none of $R^1$, $R^2$ and $R^3$ represents a non-hydrogen-bonded hydroxyl group and one of $R^4$, $R^5$ and $R^6$ represents an —OH or —SH group, with an appropriate haloalkanoic acid derivative in the presence of a suitable base,

e) production of a compound of formula I in which at least one of $R^1$, $R^2$ and $R^3$ represents alkenyloxy or alkoxy, by alkenylating or alkylating a corresponding compound of formula I in which at least one of $R^1$, $R^2$ and $R^3$ represents hydroxy,

f) production of a compound of formula I, or an ester thereof, in which at least one of $R^1$, $R^2$ and $R^3$ represents —OH and another of $R^1$, $R^2$ and $R^3$ represents an allyl group or an allyl group substituted by alkyl in the position adjacent to the benzene ring, by subjecting to an elevated temperature a corresponding compound of formula I, or an ester therefore, in which at least one of $R^1$, $R^2$ and $R^3$ represents hydrogen and another of $R^1$, $R^2$ and $R^3$ represents an allyloxy group or an alkyl substituted allyloxy group, or

g) converting an acid of formula I to a pharmaceutically acceptable derivative thereof or vice versa.

In process (a) the groups $D^4$, $D^5$ and $D^6$ may, for example, be carboxylic amide, nitrile or preferably ester, e.g. lower alkyl ester, groups, which may be hydrolysed to a —COOH or —ACOOH group. The hydrolysis may be carried out using conventional techniques, for example, under mildly basic conditions, e.g. using sodium bicarbonate. The reaction is preferably carried out at a temperature of 20° to 120°C.

In process (b) when L or Z is a reactive metal the metal may, for example, be an alkali metal, e.g. sodium, or another reactive metal, e.g. thallium. When L or Z represents a hydrocarbon chain carrying an anion forming group the anion forming group may be, for example, a halogen atom, e.g. bromine, or a sulphonate group, e.g. a methyl sulphonate or a *p*-toluenesulphonate group. When L or Z represents a hydrocarbon chain carrying a halogen atom the reaction may be carried out in the presence of a solvent which is inert under the reaction conditions, e.g. acetone and in the presence of an acid acceptor, e.g.

potassium carbonate. The reaction is also preferably carried out under anhydrous conditions and in the presence of a suitable catalyst, e.g. KI. When L or Z represents a hydrocarbon group carrying an epoxide group the reaction may be carried out at an elevated temperature in a solvent which is inert under the reaction conditions, e.g. dioxan or dimethylformamide, and in the presence of a suitable catalyst, e.g. trimethylbenzylammonium hydroxide. Alternatively the reaction may be carried out in the presence of a tertiary alcohol, e.g. *t*-butanol or 1,1-dimethyl-propan-1-ol and in the presence of the potassium salt of the alcohol as catalyst.

In process (c), hydrogenation may be effected by conventional procedures, e.g. the use of hydrogen in the presence of Raney nickel, or palladium catalyst dispersed on an inert support. The reaction is preferably carried out at a temperature of from about 20 to 80°C.

Process (d) may be conveniently effected in an appropriate solvent medium, e.g. acetone or dimethylformamide. Where one or more of $R^4$, $R^5$ and $R^6$ represent mercapto, the solvent medium may advantageously be water. The base employed is preferably an alkali-metal base, e.g. potassium carbonate or sodium hydride.

Process (e) may be carried out using, an alkenylating or alkylating agent, an alkenyl or alkyl group linked to a good leaving group, e.g. an alkenyl- or alkyl- halide. The reaction may be carried out in a solvent which is inert under the reaction conditions, e.g. dimethyl formamide, and in the presence of a base e.g. $K_2CO_3$ or NaH. The reaction is preferably carried out at a temperature of from 20° to 120°C.

In process f) the reaction may be carried out under conditions conventional for a Claisen rearrangement, e.g. at a temperature of from about 120° to 250°C optionally in a high boiling solvent which is inert under the reaction conditions, e.g. N-methylpyrrolidinone.

In process (g), the techniques involved are conventional, including treatment of a compound of formula I which contains an acidic group or a salt thereof with an appropriate base, e.g. a sodium base, or with an appropriate salt by a metathetical process.

The starting materials for the above processes are either known or may be made from known starting materials in a manner known for the production of similar known compounds.

The processes as described above may produce the compound of formula I or a derivative thereof. It is also within the scope of this invention to treat any derivative so produced to liberate the free compound of formula I, or to convert one derivative into another.

The compounds of formula I and the intermediates therefore may be isolated from their reaction mixtures using conventional techniques.

Pharmaceutically acceptable derivatives of the compounds of formula I include pharmaceutically acceptable salts, esters and amides of any —COOH group present. Suitable salts include ammonium, alkali metal (e.g. sodium, potassium and lithium) and alkaline earth metal (e.g. calcium or magnesium) salts, and salts with suitable organic bases, e.g. salts with hydroxylamine, lower alkylamines such as methylamine or ethylamine, with substituted lower alkylamines, e.g. hydroxy substituted alkylamines such as tris(hydroxymethyl)methylamine, with simple monocyclic nitrogen heterocyclic compounds. e.g. piperidine or morpholine, with an amino acid, e.g. lysine, ornithine, arginine, or an N-alkyl, especially an N-methyl derivative of any one thereof, or with an aminosugar, e.g. glucamine, N-methylglucamine or glucosamine. Suitable esters include simple lower alkyl esters, e.g. the ethyl ester, esters derived from alcohols containing basic groups, e.g. di-lower alkyl amino substituted alkanols such as the 2-(diethylamino)-ethyl ester, and acyloxy alkyl esters, e.g. a lower acyloxy-lower alkyl ester such as the pivaloyloxymethyl ester. The pharmaceutically acceptable acid addition salts of the basic esters, e.g. the hydrochloride, the hydrobromide, the maleate or the fumarate salts, may also be used. The esters may be made by conventional techniques, e.g. esterification or transesterification. The amides may be, for example, unsubstituted or mono- or di- C 1 to 6 alkyl or phenyl amides and may be made by conventional techniques, e.g. reaction of an ester of the corresponding acid with ammonia or an appropriate amine. Other pharmaceutically acceptable derivatives are compounds which will be suitable bioprecursors (prodrugs) of the compounds of formula I and will be readily apparent to those skilled in the art and may be made from the compounds of formula I using conventional processes known *per se* or by processes analogous to those described above.

The compounds of formula I and their pharmaceutically acceptable derivatives possess pharmacological properties. In particular, they are antagonists of the slow reacting substance of anaphylaxis (SRS—a) or its pathological effects, as indicated by their activity in the test described by Augstein et al, Nature New Biology, 1973, *245*, 215.

The compounds are thus indicated for use in the treatment of disorders in which SRS—A is a factor, for example skin afflictions, hay fever and obstructive airways diseases, e.g. asthma, bronchitis and bronchorrhea.

For the above mentioned uses, the dosage administered will, of course, vary depending upon the compound employed, mode of administration and treatment desired. However, in general satisfactory results are obtained when administered at a daily dosage of from about 0.05 milligrams to about 10 milligrams per kilogram of animal body weight, preferably given in divided doses 2 to 4 times a day or in substained release form. For the larger mammals, the total daily dosage is in the range of from about 1 milligram to about 700 milligrams and dosage forms suitable for administration comprise from about 12 milligrams to about 350 milligrams of the compound admixed with a solid or liquid pharmaceutical carrier or diluent. The compounds may be administered during or before the attack of the disorder to be treated.

The compounds of formula I and the pharmaceutically acceptable derivatives thereof, are more active, more stable, more selective, less toxic or possess less side effects when tested in certain pharmacological models, are more potent, have a different (e.g. longer) duration of action, have a different absorption profile (e.g. are better absorbed), are more easily formulated or possess other advantageous properties when compared to similar known compounds.

According to our invention we also provide a pharmaceutical composition comprising (preferably a minor proportion of) a compound of formula I, or a pharmaceutically acceptable derivatives thereof, in admixture with a pharmaceutically acceptable adjuvant, diluent or carrier. Examples of suitable adjuvants, diluents or carriers are:— for tablets and dragées; lactose, starch, talc or stearic acid; for capsules, tartaric acid or lactose; for suppositories, natural or hardened oils or waxes; for inhalation a coarse carrier, e.g. lactose or a compressed gas propellant, e.g. a chlorofluorohydrocarbon, and optionally a surfactant; and for topical application, wool fat, soft paraffin or a cream BP. For use in such compositions, the compound of formula I, or the pharmaceutically acceptable derivative thereof, preferably has a mass median diameter of from 0.01 to 10 microns. The compositions may also contain suitable preserving, stabilising and wetting agents, solubilizers, sweetening and colouring agents and flavourings. The compositions may, if desired, be formulated in sustained release form. We prefer compositions which are designed to be administered by inhalation.

The groups $R^1$ to $R^6$, when they contain carbon, preferably contain less than 7 carbon atoms.

The alkyl groups which $R^1$ to $R^6$ may represent preferably contain from 1 to 4 carbon atoms, a particularly preferred group being *n*-propyl.

The alkenyl groups which $R^1$ to $R^6$ may represent preferably contain from 2 to 4 carbon atoms, a particularly preferred group being allyl.

We prefer $R^1$ to $R^3$, when halogen, to be chlorine.

When one or more of $R^1$, $R^2$ and $R^3$ represents an alkanoyl group it is preferably a C 2 to 6 alkanoyl group, e.g. acetyl.

Preferably, at least one (and preferably only one) of $R^4$, $R^5$ and $R^6$ represents —COOH or —ACOOH. A particularly preferred group —ACOOH is $—OCH_2COOH$. The —COOH or —ACOOH group is preferably para to the group G.

Where one or more of $R^1$ to $R^6$ represent an alkyl group, it is preferably *ortho* to the —EXG— chain.

X preferably represents a straight chain saturated hydrocarbon group, a particularly preferred group being trimethylene or 2-hydroxytrimethylene.

We prefer E and G both to be oxygen or one to be oxygen and the other sulphur.

A preferred group of compounds of formula I are those in which E and G are both oxygen, one or more of $R^1$, $R^2$ and $R^3$ represent alkyl, alkenyl, alkoxy, alkanoyl or hydroxy and the remainder each represent hydrogen, and X, $R^4$, $R^5$ and $R^6$ are as defined above save that Y does not represent —CH=CH—.

A further preferred group of compounds of formula I are those in which E and G are both oxygen, $R^1$ represents alkanoyl C 2 to 4, $R^3$ is —OH, $R^4$ is ortho to G, $R^2$ and $R^4$, which may be the same or different, each represent alkyl C 1 to 4, $R^5$ is hydrogen and $R^6$ represents carboxy, —YCOOH or —OYCOOH in which Y does not represent —CH=CH—. In this preferred group we further prefer $R^1$ to be acetyl, $R^2$ to be *n*-propyl, $R^4$ to be *n*-propyl and $R^6$ to be $—OCH_2COOH$.

A still further preferred group of compounds of formula I are those in which proviso b) i) reads:—

i) the R group is not of formula IIIa,

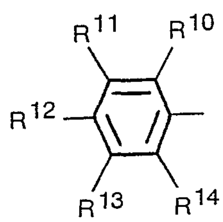

IIIa

in which

$R^{10}$ and $R^{11}$ are selected from hydrogen, halogen or alkyl

$R^{12}$ is alkanoyl,

$R^{13}$ is hydrogen or hydroxy, and

$R^{14}$ is alkyl or alkenyl,

when only one of $R^4$, $R^5$ and $R^6$ represent —COOH, —Y—COOH or —OYCOOH, or

when one of $R^4$, $R^5$ and $R^6$ represent a group —COOH, —Y—COOH or —OYCOOH and another of $R^4$, $R^5$ and $R^6$ represents —COOH.

A specifically preferred group of compounds are those of formula Ia,

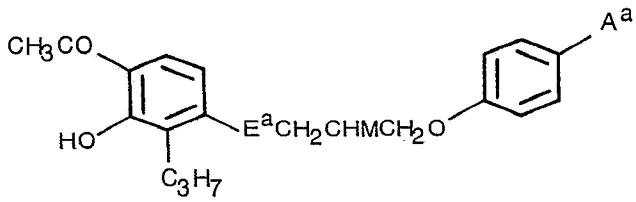

Ia

in which

M is hydrogen or —OH

$A^a$ is —$SCH_2COOH$, —CH=CHCOOH or —$CH_2CH_2COOH$, and

$E^a$ is —O— when $A^a$ is —$SCH_2COOH$ or —S— when $A^a$ is —CH=CHCOOH or —$CH_2CH_2COOH$.

Certain of the compounds of formula I possess one or more chiral centres and the invention also provides the compounds in the form of their individual optical isomers or as racemic, or other, mixtures thereof. Certain of the compounds of formula I may also exist as cis or trans isomers and in these cases the invention provides both isomeric forms. The various isomers may be prepared and/or separated using conventional processes known *per se*.

The invention is illustrated, but in no way limited, by the following Examples.

## Example 1

Sodium trans-3-[4-[3-(2-allyl-4-acetylphenoxy)propyloxy]phenyl]propenoate

(a) *Methyl trans 3-(4-[3-bromopropyloxy]phenyl) propenoate*

To a suspension of potassium carbonate (21.7 g) and 1,3-dibromopropane (145 mls) in refluxing acetone (350 mls) was added dropwise over 48 h a solution of methyl trans-4-hydroxycinnamate (25.5 g) in acetone (50 mls). The reaction mixture was allowed to cool, the potassium carbonate was removed by filtration and the solvent was evaporated. The residue was triturated with petroleum ether (bp 40—60) to afford the sub-title compound (34.6 g) mp 76—78°C.

(b) *Methyl trans-3-(4-[3-(2-allyl-4-acetylphenoxy)propyloxy]phenyl)propenoate*

A mixture of 2-allyl-4-acetyl-phenol (3.23 g) and methyl trans-3-(4-[3-bromopropyloxy]phenyl)-propenoate (5 g) in dry N,N-dimethylformamide (50 mls) containing potassium carbonate (2.54 g) was stirred for 72 hours, poured into water, and extracted with ethyl acetate. The organic solution was washed with water, dried, and evaporated to give a colourless oil which solidified on trituration with petroleum ether (bp 40—60). The crude product was purified by chromatography over silica gel to give the sub-title compound (4.67 g) mp 80—81°C.

(c) *Trans-3-(4-[3-(2-allyl-4-acetylphenoxy)propyloxy]phenyl)propenoic acid*

To a solution of the product of step b) (2 g) in dioxan (30 mls) was added 1N sodium hydroxide solution (10.16 mls) and stirring was continued for 16 hours. The solution was evaporated, the residue was dissolved in water, acidified to pH 1 and extracted with ethyl acetate. The organic solution was dried and evaporated to give a solid which was recrystallised from ethyl acetate to give the sub-title compound (1.1 g) M.pt 165—165.5°C.

*Analysis*:

| | | |
|---|---|---|
| Found: | C, 72.5; | H, 6.2% |
| $C_{23}H_{24}O_5$ Requires: | C, 72.6; | H, 6.3% |

(d) *Sodium trans-3-(4-[3-(2-allyl-4-acetylphenoxy)propyloxy]phenyl)propenoate*

The product of step c) and an equivalent amount of sodium bicarbonate were dissolved together in water and the resulting solution was freeze-dried to afford the sub-title sodium salt.

## Example 2

Sodium 3-[4-(3-[2-acetyl-3-hydroxyphenoxy]propyloxy)phenyl]propanoate

Methyl trans - 3 - [4 - (3 - [2 - acetyl - 3 - hydroxyphenoxy]propyloxy)phenyl]propenoate (1 g) in acetone (50 ml) was hydrogenated over 10% palladium on charcoal for 4 hours at atmospheric pressure. Evaporation of the solvent gave methyl 3 - [4 - (3 - [2 - acetyl - 3 - hydroxyphenoxy]propyloxy)phenyl]-propanoate (0.96 g) as an oil, characterised by 'HNMR and MS.

The oil was hydrolysed by the method in Example I (c) to give the acid of the title salt (0.62 g), mp 134—135°.

*Analysis*:

| | | |
|---|---|---|
| Found: | C, 66.6; | H, 6.2% |
| $C_{20}H_{22}O_6$ Requires: | C, 67.0; | H, 6.15% |

The title solid salt was prepared from the acid by the method of Example 1 (d).

Example 3

Sodium (4-[3-(4-acetyl-3-hydroxy-2-propylphenoxy)propoxy]phenylthio)acetate

(a) *Ethyl (4-[3-(4-acetyl-3-hydroxy-2-propylphenoxy)propoxy]phenylthio)acetate*

Ethyl (4-hydroxyphenylthio) acetate (1.5 g), potassium carbonate (1.0 g), 1 - [4 - (3 - bromopropyloxy) - 2 - hydroxy - 3 - propylphenyl]ethanone (2.25 g) and potassium iodide (0.1 g) in dry dimethylformamide (20 mls) were heated to 80°C and stirred for 18 hours under a nitrogen atmosphere. The mixture was poured into dilute (5%) hydrochloric acid and extracted with ethyl acetate which was washed with water, dried and evaporated. The resulting oil was chromatographed on silica gel using dichloromethane as eluant to give the sub-title compound as a pale yellow oil (2.3 g).

The structure was confirmed by 'H NMR and M.S.

(b) *(4-[3-(4-Acetyl-3-hydroxy-2-propylphenoxy)propoxy]phenylthio)acetic acid*

The product of step a) (2.2 g) in ethanol (25 mls) and sodium carbonate (1.5 g) in water (5 mls) were refluxed for one hour, with occasional addition of water. The mixture was poured into excess dilute hydrochloric acid and extracted into ethyl acetate, which was washed with water, dried, and evaporated to an oil. Crystallisation of the oil from ether/petroleum ether (bp 40—60) (1:3) gave the sub-title compound (0.7 g) mpt 93—94°C.

*Analysis*:

| | | | |
|---|---|---|---|
| Found: | C, 63.45; | H, 6.29; | S, 7.5% |
| $C_{22}H_{26}O_6S$ Requires: | C, 63.15; | H, 6.26; | S, 7.65% |

(c) *Sodium (4-[3-(4-acetyl-3-hydroxy-2-propylphenoxy)propoxy]phenylthio)acetate*

The product of step b) (0.6 g) was dissolved in ethanol (8 mls) and treated with an equivalent amount of 1N sodium hydroxide solution. The solvents were evaporated and the residue was dissolved in a minimum of water, filtered and freeze-dried to give the title sodium salt.

Example 4

Sodium (4-[3-(4-acetyl-3-hydroxy-2-propylphenoxy)-2-hydroxypropoxy]phenylthio)acetate

(a) *Ethyl 4-[3-(4-acetyl-3-hydroxy-2-propylphenoxy)-2-hydroxypropoxy]phenylthio)acetate*

Ethyl (4-hydroxyphenylthio)acetate (2.3 g), 1 - [4 - (2,3 - epoxypropoxy) - 2 - hydroxy - 3 - propylphenyl]ethanone (4.0 g) and benzyl trimethylammonium hydroxide (40%; 0.15 mls) in dry dimethylformamide (20 mls) were heated to 130°C for 10 hours under a nitrogen atmosphere. The mixture was poured into dilute hydrochloric acid and extracted into ethyl acetate, was washed with water, dried and evaporated to give a brown oil. Chromatography on silica gel with dichloromethane containing ethyl acetate (8%) gave the sub-title compound (2.1 g) as an oil. The structure was confirmed by 'H NMR and M.S.

(b) *Sodium (4-[3-(4-acetyl-3-hydroxy-2-propylphenoxy-2-hydroxypropoxy]phenylthio)acetate*

Hydrolysis of the product of step a) by the method of Example 3 b) gave (4 - [3 - (4 - acetyl - 3 - hydroxy - 2 - propylphenoxy - 2 - hydroxypropoxy]phenylthio)acetic acid, mp 95—96°.

*Analysis*:

| | | | |
|---|---|---|---|
| Found: | C, 60.5; | H, 5.9; | S, 7.4% |
| $C_{22}H_{26}O_7S$ Requires: | C, 60.8; | H, 6.0; | S, 7.4% |

The title salt was prepared by the method of Example 3 c).

Example 5

Sodium trans-3-(4-[3-(2-acetyl-3-allyloxyphenoxy)propyloxy]phenyl)propenoate

(a) *Methyl trans-3-(4-[3-(2-acetyl-3-allyloxyphenoxy)propyloxy]phenyl)propenoate*

A mixture of methyl trans - 3 - (4 - [3 - (2 - acetyl - 3 - hydroxyphenoxy)propyloxy]phenyl)-propenoate (4 g), dry N,N-dimethylformamide (30 mls), potassium carbonate (3.0 g), and allyl bromide (1.88 mls) was stirred for 16 hours. The mixture was poured into water and extracted with ethyl acetate. The extracts were washed with water, dried and evaporated to give the sub title compound (4.27 g), mp 73—75°C.

(b) *Sodium trans-3-(4-[3-(2-acetyl-3-allyloxyphenoxy)propyloxy]phenyl)propenoate*

Methyl trans - 3 - (4 - [3 - (2 - acetyl - 3 - allyloxyphenoxy)propyloxy]phenyl)propenoate was hydrolysed by the process of Example 1 c) to give the corresponding acid, mp 151—2°. The title salt was prepared by the process of Example 1 d).

Example 6

Sodium trans-3-(4-(3-[2-acetyl-4-allyl-3-hydroxyphenoxy)propyloxy]phenyl)propenoate

(a) *Methyl trans-3-(4-(3-[2-acetyl-4-allyl-3-hydroxyphenoxy)propyloxy]phenyl)propenoate*

Methyl trans - 3 - (4 - [3 - (2 - acetyl - 3 - allyloxyphenoxy)propyloxy]phenyl)propenoate (2 g) in N-methylpyrrolidinone (20 mls) was refluxed under a nitrogen atmosphere for 5 hours. The solution was poured into water and extracted with ethyl acetate. The extracts were dried, and evaporated to give an oil which was chromatographed on silica to give the sub-title ester (1.05 g) mp 99—100.5°.

(b) *Sodium trans-3-(4-(3-[2-acety-4-allyl-3-hydroxyphenoxy)propyloxy]phenyl)propenoate*

The product of step a) was hydrolysed by the process of Example 1 c) to give the corresponding acid, mp. 175—6°.

The title salt was prepared by the process of Example 1 d).

Example 7

By the methods of Examples 1 b), c) and d) and using appropriate starting materials were prepared the following acids and their sodium salts:—

(a) Trans-3-(4-(3-[2-propylphenoxy]propyloxy)phenyl)propenoic acid, mp 110—111°.

(b) Trans-3-(4-(3-[2-acetyl-3-hydroxyphenoxy]propyloxy)phenyl)propenoic acid, mp 239—240°.

(c) Trans 3-(4-(3-[4-acetyl-3-hydroxy-2-propylphenylthio]propyloxy)phenyl)propenoic acid, m.p. 152.5—153.5°.

(d) Trans 3-[4-(3-[3-acetyl-4-hydroxy-5-propylphenoxy]propyloxy)phenyl]propenoic acid, oil characterised by 'HNMR and MS.

Example 8

By the methods of Examples 2 and 1 d) using appropriate starting materials the following acid and its sodium salt were prepared:—

(a) 3-(4-(3-[4-Acetyl-3-hydroxy-2-propylphenythio]propyloxy)phenyl)propanoic acid, mp. 96—97°.

Example 9

By the methods of Examples 3 a), 3 b) and 1 d) using appropriate starting materials the following acids and their sodium salts were prepared:—

a) 3-[4-(5-(2-Acetyl-3-hydroxyphenoxy)pentyloxy)-3,5-diallylphenyl]propanoic acid, oil.

*Analysis*:

| | | |
|---|---|---|
| Found: | C, 72.5; | H, 7.5% |
| $C_{28}H_{34}O_6$ Requires: | C, 72.1; | H, 7.35% |

b) (4-[3-(4-Acetyl-6-bromo-3-hydroxy-2-propylphenoxy)propoxy]phenylthio)acetic acid, m.p. 109—110°.

Example 10

Sodium 6-acetyl-3-(5-phenylpentylthio)-2-propylphenoxy acetate

a) *2-Hydro-4-(5-phenylpentylthio)-3-propylacetophenone*

5-Bromopentylbenzene (1.93 g), 2-hydroxy-4-mercapto-3-propylacetophenone (1.78 g), potassium carbonate (1.28 g) and dimethylformamide (60 ml) containing potassium iodide (0.1 g) were stirred at 60° under nitrogen for 20 hours, poured into dilute hydrochloric acid and extracted with ethyl acetate. The organic phase was washed with water, dried and evaporated to yield an oil which was chromatographed over silica gel with petroleum ether (bp 40—60) containing ether (10%) to give the sub-title compound (1.85 g), mp 82—83°.

b) *Sodium 6-acetyl-3-(5-phenylpentylthio)-2-propylphenoxyacetate*

The product of step a) (0.67 g) in dry dimethylformamide (5 ml) was slowly added to stirred suspension of sodium hydride (0.09 g) in dimethylformamide (25 ml).

The solution was warmed to 35° for 0.5 hours, then cooled to 10° and treated with ethyl chloroacetate (0.2 ml). After 14 hours at room temperature the mixture was poured into dilute hydrochloric acid and extracted with ethyl acetate, which was then washed with water, dried and evaporated to an oil. Chromatography over silica with light petroleum ether (bp 40—60) containing ether (20%) gave the ester of the title compound as an oil (0.15 g). This ester was hydrolysed by the method of Example 3 b) to give, (after

crystallisation from light petroleum ether (bp 40—60) containing ether), 6 - acetyl - 3 - (5 - phenylpentylthio) - 2 - propylphenoxyacetic acid (0.8 g), mp 117—118°.

*Analysis*:

| | | | |
|---|---|---|---|
| Found: | C, 69.5; | H, 7.3; | S, 7.75% |
| $C_{24}H_{30}O_3S$ Requires: | C, 69.55; | H, 7.3; | S, 7.7% |

The title sodium salt was prepared by the method of Example 3 c).

## Example 11

Preparation of novel intermediates

a) *Methyl 3-[3,5-diallyl-4-hydroxyphenyl]propanoate*

Methyl 3-(4-allyloxyphenyl)propanoate (24.5 g) was refluxed in N-methylpyrrolidinone (100 ml) under nitrogen for 5 hours, and then poured into water. Extraction with ethyl acetate and evaporation afforded methyl 3-(3-allyl-4-hydroxyphenyl)propanoate as an oil (24.5 g), characterised by 'HNMR and MS. This oil, allyl bromide (13,3 g), and potassium carbonate (16.7 g) were heated with stirring in dimethylformamide (100 ml) at 70° under $N_2$ for 15 hours. The mixture was treated with dilute hydrochloric acid and extracted with ethyl acetate, which was then washed with 5% sodium carbonate solution, and water. Evaporation gave methyl 3-(3-allyl-4-allyloxyphenyl)propanoate as an oil (14.8 g), characterised by 'HNMR and MS. This oil was refluxed in N-methylpyrrolidinone (80 ml) for 5 hours under nitrogen, cooled, poured into water and extracted with ethyl acetate. Evaporation furnished the title compound as an oil (12.3 g), characterised by 'H NMR and MS.

b) *5-Bromo-4-(3-bromopropoxy)-2-hydroxy-3-propylacetophenone*

To 4-(3-bromopropoxy)-2-hydroxy-3-propylacetophenone (15.8 g) in glacial acetic acid (40 ml) and chloroform (20 ml) at 0° was added dropwise with stirring a solution of bromine (2.58 ml) in acetic acid (20 ml). After 10 minutes the mixture was poured into dilute sodium bisulphite solution and extracted with methylene chloride, which was washed with water, dried and evaporated to an oil. Chromatography over silica gel with petroleum ether (bp 60—80) containing ether (5%) gave the sub-title compound as an oil (12 g), characterised by 'HNMR and MS.

c) *2,5-dihydroxy-3-propylacetophenone*

5-Benzyloxy-2-hydroxyacetophenone (75.4 g), allyl bromide (41.2 g) and potassium carbonate (46.9 g) were refluxed in dimethylformamide (200 ml) for 3 hours with stirring. The mixture was poured onto ice and extracted with ether, which was then washed with 5% sodium hydroxide, and water, dried and evaporated. The residue was extracted repeatedly with hot light petroleum ether (bp 40—60). The combined extracts were concentrated, and on cooling afforded 2-allyloxy-5-benzyloxyacetophenone (29.9 g) mp 48—52°. This solid was refluxed in dimethylaniline (100 ml) under nitrogen for 11 hours, cooled, poured into dilute hydrochloric acid and extracted with ether. To the organic layer was added 20% sodium hydroxide solution (50 ml). The precipitated salt was collected, washed well with ether, and treated with dilute hydrochloric acid. Extraction and evaporation gave 3-allyl-5-benzyloxy-2-hydroxyacetophenone (22 g) as a solid. This solid in ethanol containing a little dilute hydrochloric acid was hydrogenated at 4 atmospheres and 60° over 5% palladium on charcoal for 16 hours. Evaporation of the solvent gave the subtitle compound (13 g), mp 92—93°.

**Claims for the Contracting States: BE CH DE FR GB IT LI LU NL SE**

1. Compounds of formula I,

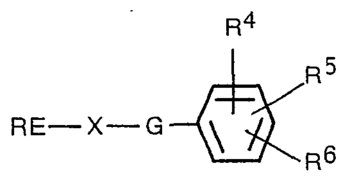

I

in which

R is a group of formula II,

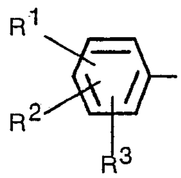

II

$R^1$, $R^2$ and $R^3$, which may be the same or different each represent hydrogen, alkyl, alkenyl, alkenyloxy, halogen, alkoxy, alkanoyl or hydroxy,

one or more of $R^4$, $R^5$ and $R^6$ represents alkyl, alkanoyl, alkenyl, —COOH or —ACOOH where A represents Y, OY or SY and Y represents a —CH=CH—, methylene, ethylene or 1,3-propylene chain, and the remainder of $R^4$, $R^5$ and $R^6$ represent hydrogen,

X represents a hydrocarbon chain having from 2 to 7 carbon atoms optionally substituted by hydroxy,

E represents —S—, —O— or —$CH_2$—, and

G represents —S— or —O—,

provided that

a) when $R^1$ and $R^2$ are hydrogen $R^3$ does not represent chlorine, isopropyl or t-butyl and is not para to E, or

b) when E and G are both —O—, either

i) the R group is not of formula III,

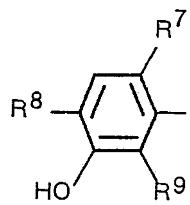

III

in which

$R^7$ is hydrogen or alkyl,

$R^8$ is alkanoyl, and

$R^9$ is alkyl or alkenyl,

when only one of $R^4$, $R^5$ and $R^6$ represent a group —COOH, —Y—COOH or —OYCOOH, or

when one of $R^4$, $R^5$ and $R^6$ represent a group —COOH, —Y—COOH or —OYCOOH and another of $R^4$, $R^5$ and $R^6$ represents —COOH, or

ii) two or more of $R^4$, $R^5$ and $R^6$ represent —ACOOH, or

iii) at least one of $R^4$, $R^5$ and $R^6$ represents a group —SYCOOH, or

iv) none of $R^4$, $R^5$ and $R^6$ represents a group selected from —COOH and —ACOOH,

c) when one of $R^4$, $R^5$ and $R^6$ represents —COOH, —$CH_2COOH$, —$(CH_2)_2$ COOH or —$(CH_2)_3$ COOH para to G, another of $R^4$, $R^5$ and $R^6$ is hydrogen or alkyl, the third of $R^4$, $R^5$ and $R^6$ is hydrogen, $R^1$ and $R^2$ are selected from hydrogen, alkyl, alkoxy and halogen, and $R^3$ is other than hydrogen, then X carries a hydroxy substituent, or

d) when $R^4$ represents —$OCH_2$ COOH, $R^5$ and $R^6$ are both hydrogen, $R^1$ is hydrogen and $R^2$ and $R^3$ are selected from alkyl, alkoxy and halogen, then X carries a hydroxy substituent,

and pharmaceutically acceptable derivatives of those compounds containing an acidic function.

2. A compound according to Claim 1, wherein each of $R^1$ to $R^6$ when they contain carbon, contain less than 7 carbon atoms.

3. A compound according to Claim 1 or 2, wherein one of $R^4$, $R^5$ and $R^6$ is —COOH or —ACOOH para to the group G.

4. A compound according to any one of the preceding claims, wherein X represents trimethylene or 2-hydroxytrimethylene.

5. A compound according to any one of the preceding claims, wherein E and G are both oxygen or one is oxygen and the other is sulphur.

6. A compound according to any one of the preceding claims, wherein E and G are both oxygen, one or more of $R^1$, $R^2$ and $R^3$ represent alkyl, alkenyl, alkoxy, alkanoyl or hydroxy and the remainder each represent hydrogen, and X, $R^4$, $R^5$ and $R^6$ are as defined in Claim 1 save that Y does not represent —CH=CH—.

7. A compound according to any one of the preceding claims, wherein E and G are both oxygen, $R^1$ represents alkanoyl C 2 to 4, $R^3$ is —OH, $R^4$ is ortho to G, $R^2$ and $R^4$, which may be the same or different, each represent alkyl C 1 to 4, $R^5$ is hydrogen and $R^6$ represents carboxy, —YCOOH or —OYCOOH in which Y does not represent —CH=CH—.

8. A compound of formula Ia,

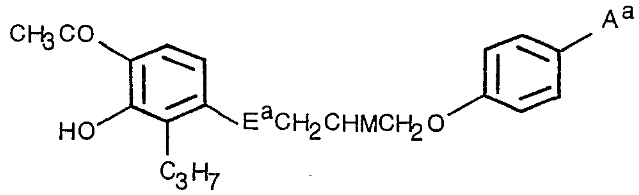

Ia

in which

M is hydrogen or —OH,

$A^a$ is $—SCH_2COOH$, —CH=CHCOOH or $—CH_2CH_2COOH$, and

$E^a$ is —O— when $A^a$ is $—SCH_2COOH$ or —S— when $A^a$ is —CH=CHCOOH or $—CH_2CH_2COOH$,

or a pharmaceutically acceptable salt thereof.

9. Trans - 3 - (4 - [3 - (2 - allyl - 4 - acetylphenoxy)propyloxy]phenyl)propenoic acid,
3 - [4 - (3 - [2 - Acetyl - 3 - hydroxyphenoxy]propyloxy)phenyl]propanoic acid,
(4 - [3 - (4 - Acetyl - 3 - hydroxy - 2 - propylphenoxy)propoxy]phenylthio)acetic acid,
(4 - [3 - (4 - acetyl - 3 - hydroxy - 2 - propylphenoxy - 2 - hydroxypropoxy]phenylthio)acetic acid,
Trans - 3 - (4 - [3 - (2 - acetyl - 3 - allyloxyphenoxy)propyloxy]phenyl)propenoic acid,
Trans - 3 - (4 - (3 - [2 - acetyl - 4 - allyl - 3 - hydroxyphenoxy)propyloxy]phenyl)propenoic acid,
Trans - 3 - (4 - (3 - [2 - propylphenoxy]propyloxy)phenyl]propenoic acid,
Trans - 3 - (4 - (3 - [2 - acetyl - 3 - hydroxyphenoxy]propyloxy)phenyl)propenoic acid,
Trans 3 - (4 - (3 - [4 - acetyl - 3 - hydroxy - 2 - propylphenylthio]propyloxy)phenyl)propenoic acid,
Trans 3 - [4 - (3 - [3 - acetyl - 4 - hydroxy - 5 - propylphenoxy]propyloxy)phenyl]propenoic acid,
3 - (4 - (3 - [4 - Acetyl - 3 - hydroxy - 2 - propylphenythio]propyloxy)phenyl)propanoic acid,
3 - [4 - (5 - (2 - Acetyl - 3 - hydroxyphenoxy)pentyloxy) - 3,5 - diallylphenyl)propanoic acid,
(4 - [3 - (4 - Acetyl - 6 - bromo - 3 - hydroxy - 2 - propylphenoxy)propoxy]phenylthio)acetic acid,
6 - Acetyl - 3 - (5 - phenylpentylthio) - 2 - propylphenoxy acetic acid,

or the sodium salt of any one thereof.

10. A pharmaceutical formulation comprising a compound according to any one of the preceding claims in admixture with a pharmaceutically acceptable adjuvant, diluent or carrier.

11. A process for the production of a compound of formula I, as defined in Claim 1, or a pharmaceutically acceptable derivative thereof, which comprises

(a) producing a compound of formula I in which one or more of $R^4$, $R^5$ and $R^6$ represents —COOH or —ACOOH, by selective hydrolysis of a compound of formula IV,

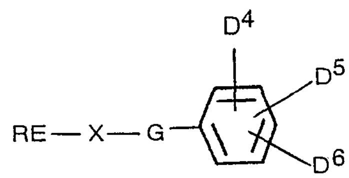

IV

in which

R, E, X and G are as defined above,

one or more of $D^4$, $D^5$ and $D^6$ represent a group hydrolysable to —COOH, or to —ACOOH, and the remainder of $D^4$, $D^5$ and $D^6$ represent groups $R^4$, $R^5$ and $R^6$ as defined above,

(b) reacting a compound of formula REZ with a compound of formula V or an ester thereof,

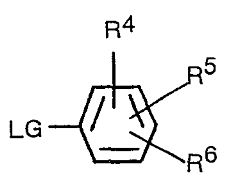

V

in which

R, E, G, $R^4$, $R^5$ and $R^6$ are as defined above, and

L and Z represent the pair of groups (i) hydrogen or a reactive metal, and (ii) a hydrocarbon chain having from 2 to 7 carbon atoms and carrying an anion forming group or an epoxide group,

(c) producing a compound of formula I in which one or more of $R^1$ to $R^6$ represent an alkyl group containing at least 2 carbon atoms and/or A represents an ethylene chain, by hydrogenation of a compound of formula I wherein one or more of $R^1$ to $R^6$ represents an alkenyl or alkanoyl group, and/or A represents —CH=CH—,

(d) producing a compound of formula I in which one or more of $R^4$, $R^5$ and $R^6$ represent —ACOOH where A represents a group OY or SY, by reacting a corresponding compound of formula I in which either an adjacent pair of $R^1$, $R^2$ and $R^3$ represent —OH and an alkanoyl group, or none of $R^1$, $R^2$ and $R^3$ represents a non-hydrogen-bonded hydroxyl group and one of $R^4$, $R^5$ and $R^6$ represents an —OH or —SH group, with an appropriate haloalkanoic acid derivative in the presence of a suitable base,

e) production of a compound of formula I in which at least one of $R^1$, $R^2$ and $R^3$ represents alkenyloxy or alkoxy, by alkenylating or alkylating a corresponding compound of formula I in which at least one of $R^1$, $R^2$ and $R^3$ represents hydroxy,

f) production of a compound of formula I, or an ester thereof, in which at least one of $R^1$, $R^2$ and $R^3$ represents —OH and another of $R^1$, $R^2$ and $R^3$ represents an allyl group or an allyl group substituted by alkyl in the position adjacent to the benzene ring, by subjecting to an elevated temperature a corresponding compound of formula I, or an ester therefore, in which at least one of $R^1$, $R^2$ and $R^3$ represents hydrogen and another of $R^1$, $R^2$ and $R^3$ represents an allyloxy group or an alkyl substituted allyloxy group, or

g) converting an acid of formula I to a pharmaceutically acceptable derivative thereof or vice versa.

**Claims for the Contracting State: AT**

1. A process for the production of a compound of formula I,

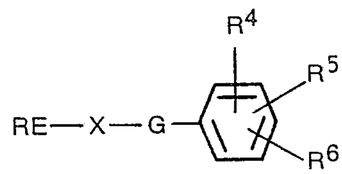

I

in which

R is a group of formula II,

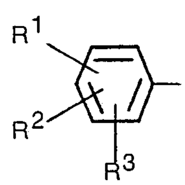

II

$R^1$, $R^2$ and $R^3$, which may be the same or different each represent hydrogen, alkyl, alkenyl, alkenyloxy, halogen, alkoxy, alkanoyl or hydroxy,

one or more of $R^4$, $R^5$ and $R^6$ represents alkyl, alkanoyl, alkenyl, —COOH or —ACOOH where A represents Y, OY or SY and Y represents a —CH=CH—, methylene, ethylene or 1,3-propylene chain, and the remainder of $R^4$, $R^5$ and $R^6$ represent hydrogen,

X represents a hydrocarbon chain having from 2 to 7 carbon atoms optionally substituted by hydroxy,

E represents —S—, —O— or —$CH_2$—, and

G represents —S— or —O—,

provided that

a) when $R^1$ and $R^2$ are hydrogen $R^3$ does not represent chlorine, isopropyl or t-butyl and is not para to E, or

b) when E and G are both —O—, either

i) the R group is not of formula III,

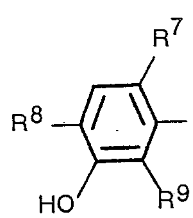

III

in which

$R^7$ is hydrogen or alkyl,

$R^8$ is alkanoyl, and

$R^9$ is alkyl or alkenyl,

when only one of $R^4$, $R^5$ and $R^6$ represent a group —COOH, —Y—COOH or —OYCOOH, or

when one of $R^4$, $R^5$ and $R^6$ represent a group —COOH, —Y—COOH or —OYCOOH and another of $R^4$, $R^5$ and $R^6$ represents —COOH, or

ii) two or more of $R^4$, $R^5$ and $R^6$ represent —ACOOH, or

iii) at least one of $R^4$, $R^5$ and $R^6$ represents a group —SYCOOH, or

iv) none of $R^4$, $R^5$ and $R^6$ represents a group selected from —COOH and —ACOOH,

c) when one of $R^4$, $R^5$ and $R^6$ represents —COOH, —$CH_2COOH$, —$(CH_2)_2$ COOH or —$(CH_2)_3$ COOH para to G, another of $R^4$, $R^5$ and $R^6$ is hydrogen or alkyl, the third of $R^4$, $R^5$ and $R^6$ is hydrogen, $R^1$ and $R^2$ are selected from hydrogen, alkyl, alkoxy and halogen, and $R^3$ is other than hydrogen, then X carries a hydroxy substituent, or

d) when $R^4$ represents —$OCH_2$ COOH, $R^5$ and $R^6$ are both hydrogen, $R^1$ is hydrogen and $R^2$ and $R^3$ are selected from alkyl, alkoxy and halogen, then X carries a hydroxy substituent,

or a pharmaceutically acceptable derivative of those compounds containing an acidic function, which comprises·

(a) producing a compound of formula I in which one or more of $R^4$, $R^5$ and $R^6$ represents —COOH or —ACOOH, by selective hydrolysis of a compound of formula IV,

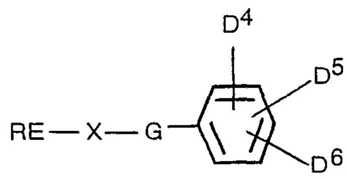

IV

in which

R, E, X and G are as defined above,

one or more of $D^4$, $D^5$ and $D^6$ represent a group hydrolysable to —COOH, or to —ACOOH, and the remainder of $D^4$, $D^5$ and $D^6$ represent groups $R^4$, $R^5$ and $R^6$ as defined above,

(b) reacting a compound of formula REZ with a compound of formula V or an ester thereof,

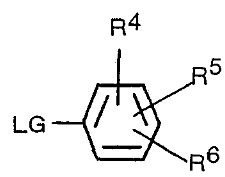

V

in which

R, E, G, $R^4$, $R^5$ and $R^6$ are as defined above, and

L and Z represent the pair of groups (i) hydrogen or a reactive metal, and (ii) a hydrocarbon chain having from 2 to 7 carbon atoms and carrying an anion forming group or an epoxide group,

(c) producing a compound of formula I in which one or more of $R^1$ to $R^6$ represent an alkyl group containing at least 2 carbon atoms and/or A represents an ethylene chain, by hydrogenation of a compound of formula I wherein one or more of $R^1$ to $R^6$ represents an alkenyl or alkanoyl group, and/or A represents —CH=CH—,

(d) producing a compound of formula I in which one or more of $R^4$, $R^5$ and $R^6$ represent —ACOOH where A represents a group OY or SY, by reacting a corresponding compound of formula I in which either an adjacent pair of $R^1$, $R^2$ and $R^3$ represent —OH and an alkanoyl group, or none of $R^1$, $R^2$ and $R^3$ represents a non-hydrogen-bonded hydroxyl group and one of $R^4$, $R^5$ and $R^6$ represents an —OH or —SH group, with an appropriate haloalkanoic acid derivative in the presence of a suitable base,

e) production of a compound of formula I in which at least one of $R^1$, $R^2$ and $R^3$ represents alkenyloxy or alkoxy, by alkenylating or alkylating a corresponding compound of formula I in which at least one of $R^1$, $R^2$ and $R^3$ represents hydroxy,

f) production of a compound of formula I, or an ester thereof, in which at least one of $R^1$, $R^2$ and $R^3$ represents —OH and another of $R^1$, $R^2$ and $R^3$ represents an allyl group or an allyl group substituted by alkyl in the position adjacent to the benzene ring, by subjecting to an elevated temperature a corresponding compound of formula I, or an ester therefore, in which at least one of $R^1$, $R^2$ and $R^3$ represents hydrogen and another of $R^1$, $R^2$ and $R^3$ represents an allyloxy group or an alkyl substituted allyloxy group, or

g) converting an acid of formula I to a pharmaceutically acceptable derivative thereof or vice versa.

2. A process according to Claim 1, wherein each of $R^1$ to $R^6$ when they contain carbon, contain less than 7 carbon atoms.

3. A process according to Claim 1 or 2, wherein one of $R^4$, $R^5$ and $R^6$ is —COOH or —ACOOH para to the group G.

4. A process according to any one of the preceding claims, wherein X represents trimethylene or 2-hydroxytrimethylene.

5. A process according to any one of the preceding claims, wherein E and G are both oxygen or one is oxygen and the other is sulphur.

6. A process according to any one of the preceding claims, wherein E and G are both oxygen, one or more of $R^1$, $R^2$ and $R^3$ represent alkyl, alkenyl, alkoxy, alkanoyl or hydroxy and the remainder each represent hydrogen, and X, $R^4$, $R^5$ and $R^6$ are as defined in Claim 1 save that Y does not represent —CH=CH—.

7. A process according to any one of the preceding claims, wherein E and G are both oxygen, $R^1$ represents alkanoyl C 2 to 4, $R^3$ is —OH, $R^4$ is ortho to G, $R^2$ and $R^4$, which may be the same or different, each represent alkyl C 1 to 4, $R^5$ is hydrogen and $R^6$ represents carboxy, —YCOOH or —OYCOOH in which Y does not represent —CH=CH—.

8. A process according to Claim 1, wherein the compound of formula I is of formula Ia,

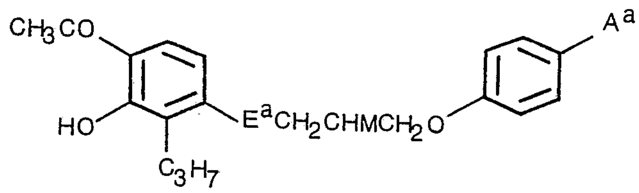

Ia

in which

M is hydrogen or —OH,

$A^a$ is $—SCH_2COOH$, —CH=CHCOOH or $—CH_2CH_2COOH$, and

$E^a$ is —O— when $A^a$ is $—SCH_2COOH$ or —S— when $A^a$ is —CH=CHCOOH or $—CH_2CH_2COOH$,

or a pharmaceutically acceptable salt thereof.

**Patentansprüche für die Vertragsstaaten: BE CH DE FR GB IT LI LU NL SE**

1. Verbindungen der Formel (I)

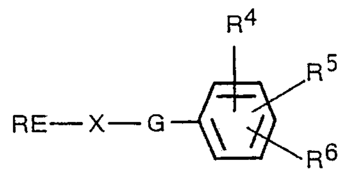

I

worin

R eine Gruppe der Formel (II)

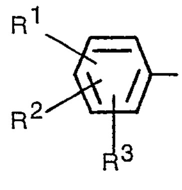

II

bedeutet, wobei

$R^1$, $R^2$ und $R^3$, die gleich oder verschieden sein können, jeweils Wasserstoff, Alkyl, Alkenyl, Alkenyloxy, Halogen, Alkoxy, Alkanoyl oder Hydroxy sind,

eines oder mehrere von $R^4$, $R^5$ und $R^6$ Alkyl, Alkanoyl, Alkenyl, —COOH oder —ACOOH bedeuten, wobei A Y, OY oder SY darstellt und Y eine —CH=CH—, Methylen-, Äthylen- oder 1,3-Propylenkette darstellt und der Rest von $R^4$, $R^5$ und $R^6$ Wasserstoff bedeutet,

X eine Kohlenwasserstoffkette mit 2 bis 7 C-Atomen, gegebenenfalls durch Hydroxy substituiert, ist,
E die Bedeutung —S—, —O— oder —$CH_2$— hat und
G —S— oder —O— darstellt,

mit der Maßgabe, daß,

a) wenn $R^1$ und $R^2$ Wasserstoff bedeuten, $R^3$ eine andere Bedeutung als Chlor, Isopropyl oder tert.Butyl hat und zu E nicht para ist, oder

b) wenn E und G beide —O— sind, entweder

i) die Gruppe R nicht die Formel (III)

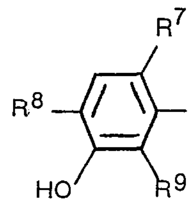

III

besitzt, worin
$R^7$ Wasserstoff oder Alkyl ist,
$R^8$ Alkanoyl bedeutet und
$R^9$ Alkyl oder Alkenyl darstellt,

wenn nur eines von $R^4$, $R^5$ und $R^6$ eine Gruppe —COOH, —Y—COOH oder —OYCOOH ist, oder

wenn eines von $R^4$, $R^5$ und $R^6$ eine Gruppe —COOH, —Y—COOH oder —OYCOOH darstellt und ein anderes von $R^4$, $R^5$ und $R^6$ —COOH ist, oder

ii) zwei oder mehr von $R^4$, $R^5$ und $R^6$ die Bedeutung —ACOOH haben, oder

iii) zumindest eines von $R^4$, $R^5$ und $R^6$ eine Gruppe —SYCOOH ist, oder

iv) keines von $R^4$, $R^5$ und $R^6$ eine Gruppe ausgewählt aus —COOH und —ACOOH darstellt,

c) wenn eines von $R^4$, $R^5$ und $R^6$ —COOH, —$CH_2$COOH, —$(CH_2)_2$COOH oder —$(CH_2)_3$COOH para zu G ist, ein anderes von $R^4$, $R^5$ und $R^6$ Wasserstoff oder Alkyl ist, das dritte von $R^4$, $R^5$ und $R^6$ Wasserstoff ist, $R^1$ und $R^2$ ausgewählt sind aus Wasserstoff, Alkyl, Alkoxy und Halogen und $R^3$ eine andere Bedeutung als Wasserstoff hat, dann X einen Hydroxysubstituenten trägt, oder

d) wenn $R^4$ —$OCH_2COOH$ ist, $R^5$ und $R^6$ beide Wasserstoff sind, $R^1$ Wasserstoff ist und $R^2$ und $R^3$ ausgewählt sind aus Alkyl, Alkoxy und Halogen, dann X einen Hydroxysubstituenten trägt,

sowie pharmazeutisch annehmbare Derivate dieser Verbindungen, die eine saure Funktion enthalten.

2. Verbindung gemäß Anspruch 1, worin jedes von $R^1$ bis $R^6$, wenn sie Kohlenstoff enthalten, weniger als 7 C-Atome enthält.

3. Verbindung gemäß Anspruch 1 oder 2, worin eines von $R^4$, $R^5$ und $R^6$ —COOH oder —ACOOH para zur Gruppe G ist.

4. Verbindung gemäß einem der vorhergehenden Ansprüche, worin X Trimethylen oder 2-Hydroxytrimethylen ist.

5. Verbindung gemäß einem der vorhergehenden Ansprüche, worin E und G beide Sauerstoff sind oder eines Sauerstoff und das andere Schwefel ist.

6. Verbindung gemäß einem der vorhergehenden Ansprüche, worin E und G beide Sauerstoff sind, eines oder mehrere von $R^1$, $R^2$ und $R^3$ Alkyl, Alkenyl, Alkoxy, Alkanoyl oder Hydroxy darstellen und der Rest jeweils Wasserstoff ist und X, $R^4$, $R^5$ und $R^6$ wie in Anspruch 1 definiert ist, mit der Maßgabe, daß Y eine andere Bedeutung als —CH=CH— hat.

7. Verbindung gemäß einem der vorhergehenden Ansprüche, worin E und G beide Sauerstoff bedeuten, $R^1$ $C_{2-4}$-Alkanoyl ist, $R^3$ —OH darstellt, $R^4$ ortho zu G ist, $R^2$ und $R^4$, die gleich oder verschieden sind; jeweils $C_{1-4}$-Alkyl darstellen, $R^5$ Wasserstoff ist und $R^6$ Carboxy, —YCOOH oder —OYCOOH bedeutet, worin Y eine andere Bedeutung als —CH=CH— hat.

8. Eine Verbindung der Formel (Ia)

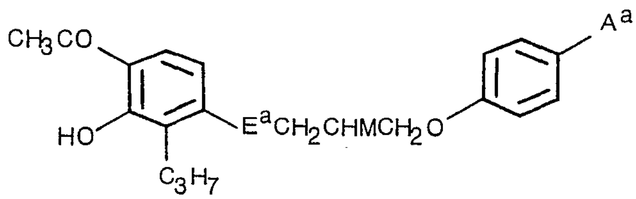

Ia

worin

M Wasserstoff oder —OH ist, $A^a$ die Bedeutung —$SCH_2COOH$, —CH=CHCOOH oder —$CH_2CH_2COOH$

hat und $E^a$ —O— ist, wenn $A^a$ —$SCH_2COOH$ ist, oder —S— ist, wenn $A^a$ —CH=CHCOOH oder —$CH_2CH_2COOH$ ist.
oder ein pharmazeutisch annehmbares Salz hievon.

9. Trans - 3 - (4 - [3 - (2 - allyl - 4 - acetylphenoxy) - propyloxy] - phenyl) - propensäure,
3 - [4 - (3 - [2 - Acetyl - 3 - hydroxyphenoxy] - propyloxy) - phenyl] - propansäure,
(4 - [3 - (4 - Acetyl - 3 - hydroxy - 2 - propylphenoxy) - propoxy] - phenylthio) - essigsäure,
(4 - [3 - (4 - Acetyl - 3 - hydroxy - 2 - propyl - phenoxy - 2 - hydroxypropoxy] - phenylthio) - essigsäure,
Trans - 3 - (4 - [3 - (2 - acetyl - 3 - allyloxyphenoxy) - propyloxy] - phenyl) - propensäure,
Trans - 3 - (4 - (3 - [2 - acetyl - 4 - allyl - 3 - hydroxyphenoxy) - propyloxy] - phenyl) - propensäure,
Trans - 3 - (4 - (3 - [2 - propylphenoxy] - propyloxy) - phenyl] - propensäure,
Trans - 3 - (4 - (3 - [2 - acetyl - 3 - hydroxyphenoxy] - propyloxy) - phenyl) - propensäure,
Trans - 3 - (4 - (3 - [4 - acetyl - 3 - hydroxy - 2 - propylphenylthio] - propyloxy) - phenyl) - propensäure,
Trans - 3 - [4 - (3 - [3 - acetyl - 4 - hydroxy - 5 - propylphenoxy] - propyloxy) - phenyl] - propensäure,
3 - (4 - (3 - [4 - Acetyl - 3 - hydroxy - 2 - propylphenylthio] - propyloxy) - phenyl) - propansäure,
3 - [4 - (5 - (2 - Acetyl - 3 - hydroxyphenoxy) - pentyloxy) - 3,5 - diallylphenyl] - propansäure,
4 - [3 - (4 - Acetyl - 6 - brom - 3 - hydroxy - 2 - propylphenoxy) - propoxy] - phenylthio) - essigsäure,
6 - Acetyl - 3 - (5 - phenylpentylthio) - 2 - propylphenoxyessigsäure
oder das Natriumsalz eines derselben.

10. Pharmazeutische Formulierung, die eine Verbindung gemäß einem der vorhergehenden Ansprüche in Mischung mit einem pharmazeutisch annehmbaren Adjuvans, Verdünnungsmittel oder Träger aufweist.

11. Verfahren zur Herstellung einer Verbindung der Formel (I), wie in Anspruch 1 definiert, oder eines pharmazeutisch annehmbaren Derivates hievon, welches umfaßt:

(a) das Herstellen einer Verbindung der Formel (I), worin eines oder mehrere von $R^4$, $R^5$ und $R^6$ —COOH oder —ACOOH bedeuten, durch selektive Hydrolyse einer Verbindung der Formel (IV)

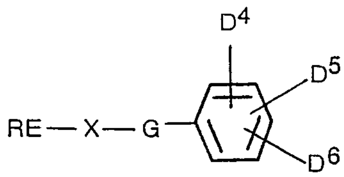

IV

worin
R, E, X und G wie oben definiert sind;
eines oder mehrere von $D^4$, $D^5$ und $D^6$ eine Gruppe darstellen, die zu —COOH oder zu —ACOOH hydrolysierbar ist, und der Rest von $D^4$, $D^5$ und $D^6$ Gruppen $R^4$, $R^5$ und $R^6$ darstellt, wie oben definiert,

(b) das Umsetzen einer Verbindung der Formel REZ mit einer Verbindung der Formel (V) oder einem Ester hievon

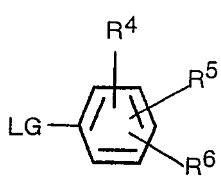

V

worin
R, E, G, $R^4$, $R^5$ und $R^6$ wie oben definiert sind und
L und Z das Paar von Gruppen (i) Wasserstoff oder ein reaktionsfähiges Metall und (ii) eine Kohlenwasserstoffkette mit 2 bis 7 C-Atomen und eine anionenbildende Gruppe oder eine Epoxidgruppe tragend darstellen,

(c) das Herstellen einer Verbindung der Formel (I), worin eines oder mehrere von $R^1$ bis $R^6$ eine Alkylgruppe mit mindestens 2 C-Atomen bedeuten und/oder A eine Äthylenkette ist, durch Hydrieren einer Verbindung der Formel (I), worin eines oder mehrere von $R^1$ bis $R^6$ eine Alkenyl- oder Alkanoylgruppe sind und/oder A die Bedeutung —CH=CH— hat,

(d) das Herstellen einer Verbindung der Formel (I), worin eines oder mehrere von $R^4$, $R^5$ und $R^6$ die Bedeutung —ACOOH haben, wobei A eine Gruppe OY oder SY ist, durch Umsetzen einer entsprechenden Verbindung der Formel (I), worin entweder ein benachbartes Paar von $R^1$, $R^2$ und $R^3$ —OH und eine Alkanoylgruppe bedeutet oder keines von $R^1$, $R^2$ und $R^3$ eine nicht an Wasserstoff gebundene Hydroxylgruppe ist und eines von $R^4$, $R^5$ und $R^6$ eine —OH— oder —SH-Gruppe darstellt, mit einem geeigneten Halogenalkansäurederivat in Anwesenheit einer geeigneten Base,

(e) das Herstellen einer Verbindung der Formel (I), worin zumindest eines von $R^1$, $R^2$ und $R^3$ Alkenyloxy oder Alkoxy darstellt, durch Alkenylieren oder Alkylieren einer entsprechenden Verbindung der Formel (I), worin zumindest eines von $R^1$, $R^2$ und $R^3$ Hydroxy bedeutet,

(f) das Herstellen einer Verbindung der Formel (I) oder eines Esters hievon, worin zumindest eines von $R^1$, $R^2$ und $R^3$ —OH bedeutet und ein anderes von $R^1$, $R^2$ und $R^3$ eine Allylgruppe oder eine Allylgruppe substituiert durch Alkyl in der dem Benzolring benachbarten Gruppe darstellt, durch Aussetzen einer entsprechenden Verbindung der Formel (I) oder eines Esters hievon, worin zumindest eines von $R^1$, $R^2$ und $R^3$ Wasserstoff bedeutet und ein anderes von $R^1$, $R^2$ und $R^3$ eine Allyloxygruppe oder eine alkylsubstituierte Allyloxygruppe darstellt, an eine erhöhte Temperatur oder

(g) das Überführen einer Säure der Formel (I) in ein pharmazeutisch annehmbares Derivat hievon oder umgekehrt.

**Patentansprüche für den Vertragsstaat: AT**

1. Verfahren zur Herstellung einer Verbindung der Formel (I)

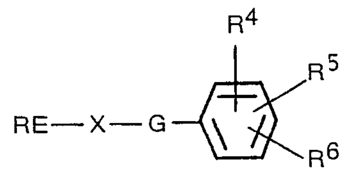

I

worin

R eine Gruppe der Formel (II)

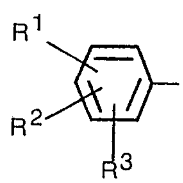

II

bedeutet, wobei

$R^1$, $R^2$ und $R^3$, die gleich oder verschieden sein können, jeweils Wasserstoff, Alkyl, Alkenyl, Alkenyloxy, Halogen, Alkoxy, Alkanoyl oder Hydroxy sind,

eines oder mehrere von $R^4$, $R^5$ und $R^6$ Alkyl, Alkanoyl, Alkenyl, —COOH oder —ACOOH bedeuten, wobei A Y, OY oder SY darstellt und Y eine —CH=CH—, Methylen-, Äthylen- oder 1,3-Propylenkette darstellt und der Rest von $R^4$, $R^5$ und $R^6$ Wasserstoff bedeutet,

X eine Kohlenwasserstoffkette mit 2 bis 7 C-Atomen, gegebenenfalls durch Hydroxy substituiert, ist,

E die Bedeutung —S—, —O— oder —$CH_2$— hat und

G —S— oder —O— darstellt,

mit der Maßgabe, daß,

a) wenn $R^1$ und $R^2$ Wasserstoff bedeuten, $R^3$ eine andere Bedeutung als Chlor, Isopropyl oder tert.Butyl hat und zu E nicht para ist, oder

b) wenn E und G beide —O— sind, entweder

i) die Gruppe R nicht die Formel (III)

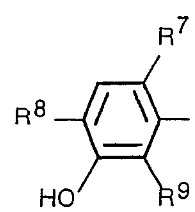

III

besitzt, worin

$R^7$ Wasserstoff oder Alkyl ist,

$R^8$ Alkanoyl bedeutet und

$R^9$ Alkyl oder Alkenyl darstellt,

wenn nur eines von $R^4$, $R^5$ und $R^6$ eine Gruppe —COOH, —Y—COOH oder —OYCOOH ist, oder

wenn eines von $R^4$, $R^5$ und $R^6$ eine Gruppe —COOH, —Y—COOH oder —OYCOOH darstellt und ein anderes von $R^4$, $R^5$ und $R^6$ —COOH ist, oder

ii) zwei oder mehr von $R^4$, $R^5$ und $R^6$ die Bedeutung —ACOOH haben, oder

iii) zumindest eines von $R^4$, $R^5$ und $R^6$ eine Gruppe —SYCOOH ist, oder

iv) keines von $R^4$, $R^5$ und $R^6$ eine Gruppe ausgewählt aus —COOH und —ACOOH darstellt,

c) wenn eines von $R^4$, $R^5$ und $R^6$ —COOH, $—CH_2COOH$, $—(CH_2)_2COOH$ oder $—(CH_2)_3COOH$ para zu G ist, ein anderes von $R^4$, $R^5$ und $R^6$ Wasserstoff oder Alkyl ist, das dritte von $R^4$, $R^5$ und $R^6$ Wasserstoff ist, $R^1$ und $R^2$ ausgewählt sind aus Wasserstoff, Alkyl, Alkoxy und Halogen und $R^3$ eine andere Bedeutung als Wasserstoff hat, dann X einen Hydroxysubstituenten trägt, oder

d) wenn $R^4$ $—OCH_2COOH$ ist, $R^5$ und $R^6$ beide Wasserstoff sind, $R^1$ Wasserstoff bedeutet und $R^2$ und $R^3$ ausgewählt sind aus Alkyl, Alkoxy und Halogen, dann X einen Hydroxysubstituenten trägt,

oder eines pharmazeutisch annehmbaren Derivates dieser Verbindungen mit einer sauren Funktion, welches umfaßt:

(a) das Herstellen einer Verbindung der Formel (I), worin eines oder mehrere von $R^4$, $R^5$ und $R^6$ —COOH oder —ACOOH bedeuten, durch selektive Hydrolyse einer Verbindung der Formel (IV)

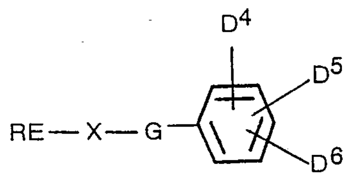

IV

worin

R, E, X und G wie oben definiert sind;

eines oder mehrere von $D^4$, $D^5$ und $D^6$ eine Gruppe darstellen, die zu —COOH oder zu —ACOOH hydrolysierbar ist, und der Rest von $D^4$, $D^5$ und $D^6$ Gruppen $R^4$, $R^5$ und $R^6$ darstellt, wie oben definiert,

(b) das Umsetzen einer Verbindung der Formel REZ mit einer Verbindung der Formel (V) oder einem Ester hievon

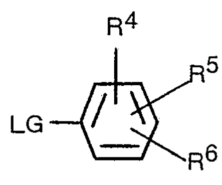

V

worin

R, E, G, $R^4$, $R^5$ und $R^6$ wie oben definiert sind und

L und Z das Paar von Gruppen (i) Wasserstoff oder ein reaktionsfähiges Metall und (ii) eine Kohlenwasserstoffkette mit 2 bis 7 C-Atomen und eine anionenbildende Gruppe oder eine Epoxidgruppe tragend darstellen,

(c) das Herstellen einer Verbindung der Formel (I), worin eines oder mehrere von $R^1$ bis $R^6$ eine Alkylgruppe mit mindestens 2 C-Atomen bedeuten und/oder A eine Äthylenkette ist, durch Hydrieren einer Verbindung der Formel (I), worin eines oder mehrere von $R^1$ bis $R^6$ eine Alkenyl- oder Alkanoylgruppe sind und/oder A die Bedeutung —CH=CH— hat,

(d) das Herstellen einer Verbindung der Formel (I), worin eines oder mehrere von $R^4$, $R^5$ und $R^6$ die Bedeutung —ACOOH haben, wobei A eine Gruppe OY oder SY ist, durch Umsetzen einer entsprechenden Verbindung der Formel (I), worin entweder ein benachbartes Paar von $R^1$, $R^2$ und $R^3$ —OH und eine Alkanoylgruppe bedeutet oder keines von $R^1$, $R^2$ und $R^3$ eine nicht an Wasserstoff gebundene Hydroxylgruppe ist und eines von $R^4$, $R^5$ und $R^6$ eine —OH— oder —SH-Gruppe darstellt, mit einem geeigneten Halogenalkansäurederivat in Anwesenheit einer geeigneten Base,

(e) das Herstellen einer Verbindung der Formel (I), worin zumindest eines von $R^1$, $R^2$ und $R^3$ Alkenyloxy oder Alkoxy darstellt, durch Alkenylieren oder Alkylieren einer entsprechenden Verbindung der Formel (I), worin zumindest eines von $R^1$, $R^2$ und $R^3$ Hydroxy bedeutet,

(f) das Herstellen einer Verbindung der Formel (I) oder eines Esters hievon, worin zumindest eines von $R^1$, $R^2$ und $R^3$ —OH bedeutet und ein anderes von $R^1$, $R^2$ und $R^3$ eine Allylgruppe oder eine Allylgruppe substituiert durch Alkyl in der dem Benzolring benachbarten Gruppe darstellt, durch Aussetzen einer entsprechenden Verbindung der Formel (I) oder eines Esters hievon, worin zumindest eines von $R^1$, $R^2$ und $R^3$ Wasserstoff bedeutet und ein anderes von $R^1$, $R^2$ und $R^3$ eine Allyloxygruppe oder eine alkylsubstituierte Allyloxygruppe darstellt, an eine erhöhte Temperatur oder

(g) das Überführen einer Säure der Formel (I) in ein pharmazeutisch annehmbares Derivat hievon oder umgekehrt.

2. Verfahren gemäß Anspruch 1, worin jedes von $R^1$ bis $R^6$, wenn sie Kohlenstoff enthalten, weniger als 7 C-Atome enthalten.

3. Verfaharen gemäß Anspruch 1 oder 2, worin eines von $R^4$, $R^5$ und $R^6$ —COOH oder —ACOOH para zur Gruppe G ist.

4. Verfahren gemäß einem der vorhergehenden Ansprüche, worin X Trimethylen oder 2-Hydroxytrimethylen ist.

5. Verfahren gemäß einem der vorhergehenden Ansprüche, worin E und G beide Sauerstoff sind oder eines Sauerstoff und das andere Schwefel ist.

6. Verfahren gemäß einem der vorhergehenden Ansprüche, worin E und G beide Sauerstoff bedeuten, eines oder mehrere von $R^1$, $R^2$ und $R^3$ Alkyl, Alkenyl, Alkoxy, Alkanoyl oder Hydroxy sind und der Rest jeweils Wasserstoff darstellt und X, $R^4$, $R^5$ und $R^6$ wie in Anspruch 1 definiert sind, mit der Maßgabe, daß Y eine andere Bedeutung als —CH=CH— hat.

7. Verfahren gemäß einem der vorhergehenden Ansprüche, worin E und G beide Sauerstoff sind, $R^1$ $C_{2-4}$-Alkanoyl bedeutet, $R^3$ —OH darstellt, $R^4$ ortho zu G ist, $R^2$ und $R^4$, die gleich oder verschieden sein können, jeweils $C_{1-4}$-Alkyl darstellen, $R^5$ Wasserstoff ist und $R^6$ Carboxy, —YCOOH oder —OYCOOH darstellt, worin Y eine andere Bedeutung als —CH=CH— hat.

8. Verfahren gemäß Anspruch 1, worin die Verbindung der Formel (I) die Formel (Ia)

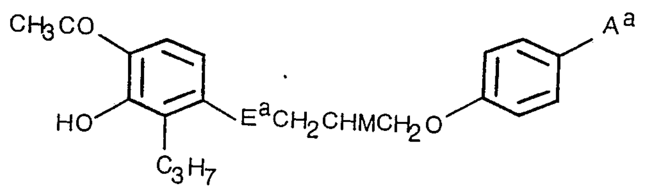

Ia

hat, worin M Wasserstoff oder —OH ist, $A^a$ die Bedeutung $—SCH_2COOH$, —CH=CHCOOH oder $—CH_2CH_2COOH$ hat und $E^a$ —O— ist, wenn $A^a$ die Bedeutung $—SCH_2COOH$ hat, oder —S— ist, wenn $A^a$ die Bedeutung —CH=CHCOOH oder $—CH_2CH_2COOH$ hat, oder ein pharmazeutisch annehmbares Salz hievon.

**Revendications pour les Etats contractants: BE CH DE FR GB IT LI LU NL SE**

1. Composés de formule I,

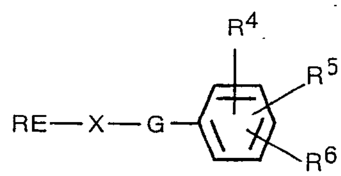

I

où

R représente un radical de formule II,

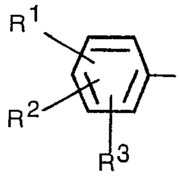

II

$R^1$, $R^2$ et $R^3$, qui peuvent être identiques ou différents, représentent chacun un atome d'hydrogène ou d'halogène ou radical alcoyle, alcényle, alcényloxy, alcoxy, alcanoyle ou hydroxyle,

l'un ou plusieurs d'entre $R^4$, $R^5$ et $R^6$ représentent un radical alcoyle, alcanoyle, alcényle, —COOH ou —ACOOH, où A représente Y, OY ou SY et Y représente une chaîne —CH=CH—, méthylène, éthylène ou 1,3-propylène, et le ou les autres d'entre $R^4$, $R^5$ et $R^6$ représentent un ou des atomes d'hydrogène,

X représente une chaîne hydrocarbonée comptant 2 à 7 atomes de carbone éventuellement substituée par hydroxyle,

E représente —S—, —O— ou $—CH_2—$, et

G représente —S— ou —O—,

avec la restriction que

a) lorsque $R^1$ et $R^2$ représentent des atomes d'hydrogène, $R^3$ ne représente pas un atome de chlore ou radical isopropyle ou t-butyle et n'occupe pas la position para par rapport à E, ou

b) lorsque E et G représentent tous deux —O—, soit

i) le radical R n'est pas de formule III,

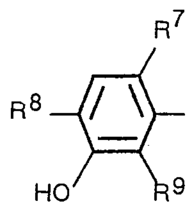

III

où

$R^7$ représente un atome d'hydrogène ou radical alcoyle,

$R^8$ représente un radical alcanoyle et

$R^9$ représente un radical alcoyle ou alcényle,

lorsqu'un seul d'entre $R^4$, $R^5$ et $R^6$ représente un radical —COOH, —Y—COOH ou —OYCOOH, ou

lorsque l'un d'entre $R^4$, $R^5$ et $R^6$ représente un radical —COOH, —Y—COOH ou —OYCOOH et un autre d'entre $R^4$, $R^5$ et $R^6$ représente un radical —COOH, soit

ii) deux ou davantage d'entre $R^4$, $R^5$ et $R^6$ représentent des radicaux —ACOOH, soit

iii) au moins l'un d'entre $R^4$, $R^5$ et $R^6$ représente un radical —SYCOOH, soit

iv) aucun d'entre $R^4$, $R^5$ et $R^6$ ne représente un radical choisi entre —COOH et —ACOOH,

c) lorsque l'un d'entre $R^4$, $R^5$ et $R^6$ représente un radical —COOH, $—CH_2COOH$, $—(CH_2)_2COOH$ ou $—(CH_2)_3COOH$ en para par rapport à G, un autre d'entre $R^4$, $R^5$ et $R^6$ représente un atome d'hydrogène ou radical alcoyle, le troisième d'entre $R^4$, $R^5$ et $R^6$ représente un atome d'hydrogène, $R^1$ et $R^2$ sont choisis entre les atomes d'hydrogène et d'halogène et radicaux alcoyle et alcoxy et $R^3$ représente autre chose qu'un atome d'hydrogène, alors X porte un substituant hydroxyle ou

d) lorsque $R^4$ représente un radical $—OCH_2COOH$, $R^5$ et $R^6$ représentent tous deux des atomes d'hydrogène, $R^1$ représente un atome d'hydrogène et $R^2$ et $R^3$ sont choisis entre les radicaux alcoyle, alcoxy et atomes d'halogène, alors X porte un substituant hydroxyle,

et les dérivés pharmaceutiquement acceptables de ceux de ces composés contenant une fonction acide.

2. Composé suivant la revendication 1, dans lequel chacun d'entre $R^1$ à $R^6$, lorsqu'ils contiennent du carbone, compte moins de 7 atomes de carbone.

3. Composé suivant la revendication 1 ou 2, dans lequel l'un d'entre $R^4$, $R^5$ et $R^6$ représente un radical —COOH ou —ACOOH en para par rapport au radical G.

4. Composé suivant l'une quelconque des revendications précédentes, dans lequel X représente un radical triméthylène ou 2-hydroxytriméthylène.

5. Composé suivant l'une quelconque des revendications précédentes, dans lequel E et G représentent tous deux des atomes d'oxygène ou l'un représente un atome d'oxygène et l'autre un atome de soufre.

6. Composé suivant l'une quelconque des revendications précédentes, dans lequel E et G représentent tous deux des atomes d'oxygène, l'un ou plusieurs d'entre $R^1$, $R^2$ et $R^3$ représentent un radical alcoyle, alcényle, alcoxy, alcanoyle ou hydroxyle et les autres représentent chacun un atome d'hydrogène et X, $R^4$, $R^5$ et $R^6$ sont tels que définis dans la revendication 1, sauf que Y ne représente pas —CH=CH—.

7. Composé suivant l'une quelconque des revendications précédentes, dans lequel E et G représentent tous deux des atomes d'oxygène, $R^1$ représente un radical alcanoyle en C 2 à 4, $R^3$ représente —OH, $R^4$ est en ortho par rapport à G, $R^2$ et $R^4$, qui peuvent être identiques ou différents, représentent chacun un radical alcoyle en C 1 à 4, $R^5$ représente un atome d'hydrogène et $R^6$ représente un radical carboxyle, YCOOH ou —OYCOOH où Y ne représente pas —CH=CH—.

8. Composé de formule Ia,

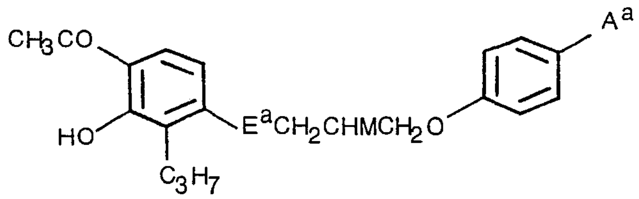

Ia

où

M représente un atome d'hydrogène ou radical —OH,

$A^a$ représente —$SCH_2COOH$, —CH=CHCOOH ou —$CH_2CH_2COOH$, et

$E^a$ représente —O— lorsque $A^a$ représente —$SCH_2COOH$ ou bien —S— lorsque $A^a$ représente —CH=CHCOOH ou —$CH_2CH_2COOH$,

ou un sel pharmaceutiquement acceptable de celui-ci.

9. L'acide trans - 3 - (4 - [3 - (2 - allyl - 4 - acétylphénoxy)propyloxy]phényl)propénoïque,

l'acide 3 - [4 - (3 - [2 - acétyl - 3 - hydroxyphénoxy]propyloxy)phényl]propanoïque,

l'acide (4 - [3 - (4 - acétyl - 3 - hydroxy - 2 - propylphénoxy)propoxy]phénylthio)acétique,

l'acide (4 - [3 - (4 - acétyl - 3 - hydroxy - 2 - propylphénoxy) - 2 - hydroxypropoxy]phénylthio)acétique,

l'acide trans - 3 - (4 - [3 - (2 - acétyl - 3 - allyloxyphénoxy)propyloxy]phényl)propénoïque,

l'acide trans - 3 - (4 - (3 - [2 - acétyl - 4 - allyl - 3 - hydroxyphénoxy)propyloxy]phényl)propénoïque,

l'acide trans - 3 - (4 - (3 - [2 - propylphénoxy]propyloxy)phényl]propénoïque,

l'acide trans - 3 - (4 - (3 - [2 - acétyl - 3 - hydroxyphénoxy]propyloxy)phényl)propénoïque,

l'acide trans - 3 - (4 - (3 - [4 - acétyl - 3 - hydroxy - 2 - propylphénylthio]propyloxy)phényl)propénoïque,

l'acide trans - 3 - [4 - (3 - [3 - acétyl - 4 - hydroxy - 5 - propylphénoxy]propyloxy)phényl]propénoïque,

l'acide 3 - (4 - (3 - [4 - acétyl - 3 - hydroxy - 2 - propylphénylthio]propyloxy)phényl)propanoïque,

l'acide 3 - [4 - (5 - (2 - acétyl - 3 - hydroxyphénoxy)pentyloxy)3,5-diallylphényl]propanoïque,

l'acide (4 - [3 - (4 - acétyl - 6 - bromo - 3 - hydroxy - 2 - propylphénoxy)propoxy]phénylthio)acétique,

l'acide 6 - acétyl - 3 - (5 - phénylpentylthio) - 2 - propylphénoxyacétique,

ou le sel de sodium de l'un quelconque de ces acides.

10. Composition pharmaceutique, comprenant un composé suivant l'une quelconque des revendications précédentes, en mélange avec un adjuvant, diluant ou excipient pharmaceutiquement acceptable.

11. Procédé de production d'un composé de formule I tel que défini dans la revendication 1, ou d'un dérivé pharmaceutiquement acceptable de celui-ci, qui comprend

(a) la production d'un composé de formule I où l'un ou plusieurs d'entre $R^4$, $R^5$ et $R^6$ représentent des radicaux —COOH ou —ACOOH, par hydrolyse sélective d'un composé de formule IV,

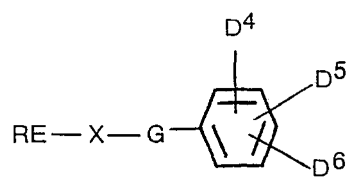

IV

où

R, E, X et G sont tels que définis ci-dessus,

l'un ou plusieurs d'entre $D^4$, $D^5$ et $D^6$ représentent un radical hydrolysable en —COOH ou en —ACOOH et le ou les autres d'entre $D^4$, $D^5$ et $D^6$ représentent un ou des radicaux $R^4$, $R^5$ et $R^6$ tels que définis ci-dessus,

(b) la réaction d'un composé de formule REZ avec un composé de formule V ou un ester de celui-ci,

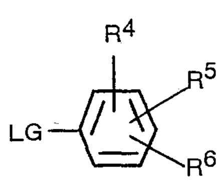

V

où

R, E, G, $R^4$, $R^5$ et $R^6$ sont tels que définis ci-dessus, et

L et Z représentent la paire de radicaux (i) atome d'hydrogène ou de métal réactif et (ii) une chaîne hydrocarbonée comptant 2 à 7 atomes de carbone et portant un radical formant anion ou un radical époxyde,

(c) la production d'un composé de formule I où l'un ou plusieurs d'entre $R^1$ à $R^6$ représentent un radical alcoyle comptant au moins 2 atomes de carbone et/ou A représente une chaîne éthylène, par hydrogénation d'un composé de formule I où l'un ou plusieurs d'entre $R^1$ à $R^6$ représentent un radical alcényle ou alcanoyle et/ou A représente —CH=CH—,

(d) la production d'un composé de formule I où l'un ou plusieurs d'entre $R^4$, $R^5$ et $R^6$ représentent un radical —ACOOH où A représente un radical OY ou SY, par réaction d'un composé correspondant de formule I ou soit une paire adjacente d'entre $R^1$, $R^2$ et $R^3$ représente un radical —OH et un radical alcanoyle, soit aucun d'entre $R^1$, $R^2$ et $R^3$ ne représente un radical hydroxyle exempt de liaison hydrogène et l'un d'entre $R^4$, $R^5$ et $R^6$ représente un radical —OH ou —SH, avec un dérivé d'acide halogéno-alcanoïque approprié en présence d'une base appropriée,

(e) la production d'un composé de formule I où au moins l'un d'entre $R^1$, $R^2$ et $R^3$ représente un radical alcényloxy ou alcoxy, par alcénylation ou alcoylation d'un composé correspondant de formule I où au moins l'un d'entre $R^1$, $R^2$ et $R^3$ représente un radical hydroxyle,

(f) la production d'un composé de formule I, ou d'un ester de celui-ci, où au moins l'un d'entre $R^1$, $R^2$ et $R^3$ représente un radical —OH et un autre d'entre $R^1$, $R^2$ et $R^3$ représente un radical allyle ou un radical allyle substitué par alcoyle en la position adjacente au cycle benzénique, par exposition à une température élevée d'un composé correspondant de formule I, ou d'un ester de celui-ci, où au moins l'un d'entre $R^1$, $R^2$ et $R^3$ représente un atome d'hydrogène et un autre d'entre $R^1$, $R^2$ et $R^3$ représente un radical allyloxy ou un radical allyloxy alcoyl-substitué, ou

(g) la conversion d'un acide de formule I en un dérivé pharmaceutiquement acceptable de celui-ci, et réciproquement.

**Revendications pour l'Etat contractant: AT**

1. Procédé de production d'un composé de formule I,

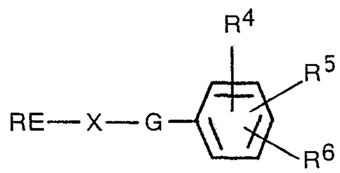

I

où

R représente un radical de formule II,

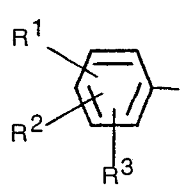

II

$R^1$, $R^2$ et $R^3$, qui peuvent être identiques ou différents, représentent chacun un atome d'hydrogène ou d'halogène ou radical alcoyle, alcényle, alcényloxy, alcoxy, alcanoyle ou hydroxyle,

l'un ou plusieurs d'entre $R^4$, $R^5$ et $R^6$ représentent un radical alcoyle, alcanoyle, alcényle, —COOH ou —ACOOH, où A représente Y, OY ou SY et Y représente une chaîne —CH=CH—, méthylène, éthylène ou 1,3-propylène, et le ou les autres d'entre $R^4$, $R^5$ et $R^6$ représentent un ou des atomes d'hydrogène,

X représente une chaîne hydrocarbonée comptant 2 à 7 atomes de carbone éventuellement substituée par hydroxyle,

E représente —S—, —O— ou —$CH_2$—, et

G représente —S— ou —O—,

avec la restriction que

a) lorsque $R^1$ et $R^2$ représentent des atomes d'hydrogène, $R^3$ ne représente pas un atome de chlore ou radical isopropyle ou t-butyle et n'occupe pas la position para par rapport à E, ou

b) lorsque E et G représentent tous deux —O—, soit

i) le radical R n'est pas de formule III,

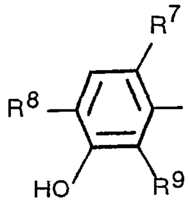

III

où

$R^7$ représente un atome d'hydrogène ou radical alcoyle,

$R^8$ représente un radical alcanoyle et

$R^9$ représente un radical alcoyle ou alcényle,

lorsqu'un seul d'entre $R^4$, $R^5$ et $R^6$ représente un radical —COOH, —Y—COOH ou —OYCOOH, ou

lorsque l'un d'entre $R^4$, $R^5$ et $R^6$ représente un radical —COOH, —Y—COOH ou —OYCOOH et un autre d'entre $R^4$, $R^5$ et $R^6$ représente un radical —COOH, soit

ii) deux ou davantage d'entre $R^4$, $R^5$ et $R^6$ représentent des radicaux —ACOOH, soit

iii) au moins l'un d'entre $R^4$, $R^5$ et $R^6$ représente un radical —SYCOOH, soit

iv) aucun d'entre $R^4$, $R^5$ et $R^6$ ne représente un radical choisi entre —COOH et —ACOOH,

c) lorsque l'un d'entre $R^4$, $R^5$ et $R^6$ représente un radical —COOH, $-CH_2COOH$, $-(CH_2)_2COOH$ ou $-(CH_2)_3COOH$ en para par rapport à G, un autre d'entre $R^4$, $R^5$ et $R^6$ représente un atome d'hydrogène ou radical alcoyle, le troisième d'entre $R^4$, $R^5$ et $R^6$ représente un atome d'hydrogène, $R^1$ et $R^2$ sont choisis entre les atomes d'hydrogène et d'halogène et radicaux alcoyle et alcoxy et $R^3$ représente autre chose qu'un atome d'hydrogène, alors X porte un substituant hydroxyle ou

d) lorsque $R^4$ représente un radical $-OCH_2COOH$, $R^5$ et $R^6$ représentent tous deux des atomes d'hydrogène, $R^1$ représente un atome d'hydrogène et $R^2$ et $R^3$ sont choisis entre les radicaux alcoyle, alcoxy et atomes d'halogène, alors X porte un substituant hydroxyle,

ou d'un dérivé pharmaceutiquement acceptable de ceux de ces composés contenant une fonction acide, qui comprend

(a) la production d'un composé de formule I où l'un ou plusieurs d'entre $R^4$, $R^5$ et $R^6$ représentent des radicaux —COOH ou —ACOOH, par hydrolyse sélective d'un composé de formule IV,

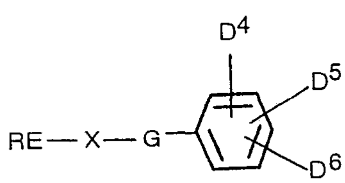

IV

où

R, E, X et G sont tels que définis ci-dessus,

l'un ou plusieurs d'entre $D^4$, $D^5$ et $D^6$ représentent un radical hydrolysable en —COOH ou en —ACOOH et le ou les autres d'entre $D^4$, $D^5$ et $D^6$ représentent un ou des radicaux $R^4$, $R^5$ et $R^6$ tels que définis ci-dessus,

(b) la réaction d'un composé de formule REZ avec un composé de formule V ou un ester de celui-ci,

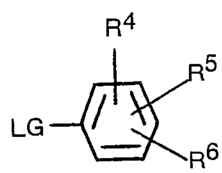

V

où

R, E, G, $R^4$, $R^5$ et $R^6$ sont tels que définis ci-dessus, et

L et Z représentent la paire de radicaux (i) atome d'hydrogène ou de métal réactif et (ii) une chaîne hydrocarbonée comptant 2 à 7 atomes de carbone et portant un radical formant anion ou un radical époxyde,

(c) la production d'un composé de formule I où l'un ou plusieurs d'entre $R^1$ à $R^6$ représentent un radical alcoyle comptant au moins 2 atomes de carbone et/ou A représente une chaîne éthylène, par hydrogénation d'un composé de formule I où l'un ou plusieurs d'entre $R^1$ à $R^6$ représentent un radical alcényle ou alcanoyle et/ou A représente —CH=CH—,

(d) la production d'un composé de formule I où l'un ou plusieurs d'entre $R^4$, $R^5$ et $R^6$ représentent un radical —ACOOH où A représente un radical OY ou SY, par réaction d'un composé correspondant de formule I ou soit une paire adjacente d'entre $R^1$, $R^2$ et $R^3$ représente un radical —OH et un radical alcanoyle, soit aucun d'entre $R^1$, $R^2$ et $R^3$ ne représente un radical hydroxyle exempt de liaison hydrogène et l'un d'entre $R^4$, $R^5$ et $R^6$ représente un radical —OH ou —SH, avec un dérivé d'acide halogéno-alcanoïque approprié en présence d'une base appropriée,

(e) la production d'un composé de formule I où au moins l'un d'entre $R^1$, $R^2$ et $R^3$ représente un radical alcényloxy ou alcoxy, par alcénylation ou alcoylation d'un composé correspondant de formule I où au moins l'un d'entre $R^1$, $R^2$ et $R^3$ représente un radical hydroxyle,

(f) la production d'un composé de formule I, ou d'un ester de celui-ci, où au moins l'un d'entre $R^1$, $R^2$ et $R^3$ représente un radical —OH et un autre d'entre $R^1$, $R^2$ et $R^3$ représente un radical allyle ou un radical allyle substitué par alcoyle en la position adjacente au cycle benzénique, par exposition à une température élevée d'un composé correspondant de formule I, ou d'un ester de celui-ci, où au moins l'un d'entre $R^1$, $R^2$ et $R^3$ représente un atome d'hydrogène et un autre d'entre $R^1$, $R^2$ et $R^3$ représente un radical allyloxy ou un radical allyloxy alcoyl-substitué, ou

(g) la conversion d'un acide de formule I en un dérivé pharmaceutiquement acceptable de celui-ci, et réciproquement.

2. Procédé suivant la revendication 1, dans lequel chacun d'entre $R^1$ à $R^6$, lorsqu'ils contiennent du carbone, compte moins de 7 atomes de carbone.

3. Procédé suivant la revendication 1 ou 2, dans lequel l'un d'entre $R^4$, $R^5$ et $R^6$ représente un radical —COOH ou —ACOOH en para par rapport au radical G.

4. Procédé suivant l'une quelconque des revendications précédentes, dans lequel X représente un radical triméthylène ou 2-hydroxytriméthylène.

5. Procédé suivant l'une quelconque des revendications précédentes, dans lequel E et G représentent tous deux des atomes d'oxygène ou l'un représente un atome d'oxygène et l'autre un atome de soufre.

6. Procédé suivant l'une quelconque des revendications précédentes, dans lequel E et G représentent tous deux des atomes d'oxygène, l'un ou plusieurs d'entre $R^1$, $R^2$ et $R^3$ représentent un radical alcoyle, alcényle, alcoxy, alcanoyle ou hydroxyle et les autres représentent chacun un atome d'hydrogène et X, $R^4$, $R^5$ et $R^6$ sont tels que définis dans la revendication 1, sauf que Y ne représente pas —CH=CH—.

7. Procédé suivant l'une quelconque des revendications précédentes, dans lequel E et G représentent tous deux des atomes d'oxygène, $R^1$ représente un radical alcanoyle en C 2 à 4, $R^3$ représente —OH, $R^4$ est en ortho par rapport à G, $R^2$ et $R^4$, qui peuvent être identiques ou différents, représentent chacun un radical alcoyle en C 1 à 4, $R^5$ représente un atome d'hydrogène et $R^6$ représente un radical carboxyle, YCOOH ou —OYCOOH où Y ne représente pas —CH=CH—.

8. Procédé suivant la revendication 1, dans lequel le composé de formule I est de formule Ia,

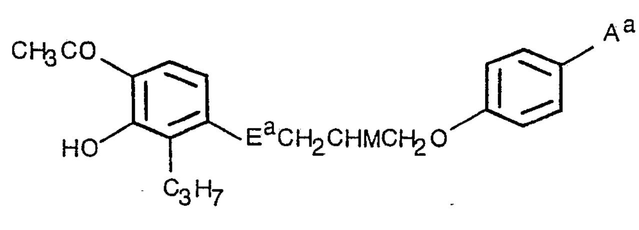

Ia

où

M représente un atome d'hydrogène ou radical —OH,

$A^a$ représente —$SCH_2COOH$, —CH=CHCOOH ou —$CH_2CH_2COOH$, et

$E^a$ représente —O— lorsque $A^a$ représente —$SCH_2COOH$ ou bien —S— lorsque $A^a$ représente —CH=CHCOOH ou —$CH_2CH_2COOH$,

ou un sel pharmaceutiquement acceptable de celui-ci.